# EUROPÄISCHE PATENTSCHRIFT

(11) **EP 0 600 911 B1**
(45) Veröffentlichungstag und Bekanntmachung des Hinweises auf die Patenterteilung: **13.03.1996**
(21) Anmeldenummer: 92915632.1
(22) Anmeldetag: 22.07.1992
(51) Int. Cl.: A61B 8/08, A61C 19/04

(54) **MESSVERFAHREN FÜR DIE SCHLEIMHAUTDICKE EINES KIEFERKNOCHENKAMMS**
PROCESS MEASURING THE THICKNESS OF THE MUCOUS MEMBRANE OF A JAWBONE RIDGE
PROCEDE SERVANT A MESURER L'EPAISSEUR DE LA MUQUEUSE DE LA CRETE D'UNE MACHOIRE

(30) Priorität: 01.08.1991 DE 4125503
(43) Veröffentlichungstag der Anmeldung: 15.06.1994
(73) Patentinhaber: KRAUTKRÄMER GmbH & Co., D-50354 Hürth (DE); Krupp Medizintechnik GmbH, D-45021 Essen (DE)
(72) Erfinder: KNAPP, Gerd, H., D-8000 München 5 (DE); POLLOCK, Benedikt, D-4712 Werne (DE); BONENKAMP, Heiko, D-5250 Engelskirchen (DE); VOLKMANN, Klaus, D-5060 Bergisch Gladbach 2 (DE)
(74) Vertreter: Bauer, Wulf, Dr.
(86) Internationale Anmeldenummer: DE9200593
(87) Internationale Veröffentlichungsnummer: WO9302623

(56) Entgegenhaltungen:
- EP-A- 0 305 519
- EP-A- 0 353 209
- US-A- 4 603 702
- US-A- 4 674 517

## Beschreibung

Die Erfindung bezieht sich auf ein Meßverfahren für die Schleimhautdicke eines Kieferknochenkamms. Zahnärzte benötigen eine Messung der Schleimhautdicke, um vor einer Implantation von Schraubkernen, beispielsweise Titanschraubkernen in einen Kieferknochen die eigentliche Dicke des Kieferknochens feststellen zu können, damit Sicherheit besteht, daß genügend Knochensubstanz für eine tragfähige Implantation vorhanden ist. Nach dem Stand der Technik wird die Schleimhautdicke mit einem Eindringkörper (Nadel) bestimmt, hierzu wird die Schleimhaut durchstochen und festgestellt, welchen Weg die Nadel zurücklegt, bis sie auf den Kieferknochen stößt. Dieses Verfahren ist nicht nur schmerzhaft, sondern birgt auch ein Infektionsrisiko.

Aus der EP-A-0 353 209 ist eine Vorrichtung für die Inspektion von Zähnen mittels Ultraschall bekannt, bei der zu überprüfende Zähne mittels eines Arrays von Ultraschallschwingern abgetastet werden. Zwischen dem Array und dem Zahnfleisch wird ein Vorlaufkörper angeordnet, der u.a. im wesentlichen aus Wasser bestehen kann.

US-A-4 603 702 zeigt eine Schaltung, mit der gemessen werden kann, wie lange ein Patient einer Ultraschallstrahlung ausgesetzt ist. Die Schaltung kann unterscheiden, ob ein Ultraschallschwinger an einen Patienten oder an Luft angekoppelt ist. Die Messung der Bestrahlungszeit erfolgt für die Dauer der Ankoppelung des Ultraschallschwingers an einen Patienten.

Die Erfindung hat es sich zur Aufgabe gemacht, ein Meßverfahren anzugeben, bei dem ohne Verletzung der Schleimhaut gearbeitet werden kann.

Diese Aufgabe wird gelöst durch das Verfahren nach Anspruch 1.

Das erfindungsgemäße Verfahren arbeitet mit Ultraschall nach dem Impuls-Echo-Verfahren, auch Impuls-Reflexions-Methode genannt. Zu Ultraschallmeßverfahren allgemein wird auf das DE-Buch J. Krautkrämer und H. Krautkrämer "Werkstoffprüfung mit Ultraschall" 4. Aufl., Springer Verlag verwiesen. Von dem im Sondengehäuse der Meßvorrichtung angeordneten Ultraschallschwinger werden periodisch Ultraschallimpulse ausgesandt, die den Vorlaufkörper durchlaufen und von dessen Koppelfläche in die Schleimhaut eintreten. Die Impulse werden am Übergang (Grenzfläche) zwischen Schleimhaut und Kieferknochenoberfläche reflektiert. Aus der Schallaufzeit wird über die bekannte Schallgeschwindigkeit (etwa 1500 m/s) die Schleimhautdicke ermittelt.

Eine qualitativ gute Ankopplung der Koppelfläche an die Schleimhaut wird dadurch erreicht, daß der Vorlaufkörper aus einem Material gefertigt ist, das eine ähnliche Schallimpedanz hat wie die Schleimhaut. Diese ist ein Gewebe und hat damit bei Ultraschall im wesentlichen die Eigenschaften von Wasser. Ist der Vorlaufkörper in gutem Kontakt mit der Schleimhaut, stellt sich kein oder nur ein kleiner Sprung (Schallhärtesprung) an der Grenzfläche Koppelfläche/Schleimhautoberfläche in der Schallimpedanz ein mit der Folge, daß dort praktisch keine Schallreflexion stattfindet. Das Kriterium geringer oder fehlender Schallreflexion am Übergang zwischen Koppelfläche und Schleimhaut wird benutzt, um zwischen guter und schlechter Ankopplung zu unterscheiden. Eine Messung wird nur dann durchgeführt, wenn die Ankopplung gut, also das Echo der Grenzfläche Koppelfläche/Schleimhautoberfläche unter einem Schwellenwert liegt.

Um dabei sicherzustellen, daß das Echo von der Koppelfläche normalerweise vorliegt, wird während einer Kalibrierungsphase, bei der die Koppelfläche frei ist und nur an Luft angrenzt, dieses Echo erfaßt. Es muß eine gewisse Amplitude aufweisen. Dabei wird nicht nur ein Echo, nämlich vorzugsweise das erste Echo der Koppelfläche, während der Kalibrierungsphase erfasst und bewertet, sondern es wird auch ein nachfolgendes, vorzugsweise das zweite Echo der Koppelfläche, ebenso ausgewertet. Während der Kalibrierungsphase soll die Koppelfläche völlig frei sein, sie darf nicht mit Wassertropfen oder Schmutz belegt sein, sondern ausschließlich Luft gegenüberstehen. Ist sie jedoch irgendwie belegt oder bedeckt, so äußert sich dies darin, daß das Verhältnis von zwei aufeinanderfolgenden Echos verändert ist. Das Verhältnis zweier Echos wird also erfindungsgemäß dazu benutzt, um eine Aussage zu erhalten, daß während der Kalibrierungsphase die Koppelfläche tatsächlich frei ist.

In einer Weiterbildung wird zugleich die Amplitude des Echos der Koppelfläche ausgenutzt, um die Sendeenergie zu steuern. Die dem Ultraschallschwinger periodisch zugesandte elektrische Energie wird so bemessen, daß das Echo der Koppelfläche eine gewisse Amplitude aufweist. Diese Steuerung der Sendeenergie hat den Vorteil, daß die Steuerschaltung für die Sendeenergie nicht die mangelnde Schallreflexion an der Koppelfläche durch Erhöhen der Sendeenergie ausgleicht. Es findet nur dann eine eigentliche Messung statt, wenn in der Kalibrierungsphase aufeinanderfolgende Echos von der Koppelfläche nicht so weit voneinander abweichen, daß ein (zweiter) Schwellenwert unterschritten wird. Wird er jedoch unterschritten, wird ein Error-Signal ausgegeben und die eigentliche Messung gesperrt.

Als vorteilhaft hat es sich erwiesen, wenn die Auswerteelektronik des Auswertegerätes akustische Signale für schlechte Ankopplung und für eine erfolgreich durchgeführte Messung abgibt. Beim praktischen Einsatz des Meßgerätes muß der Bedienende alle Konzentration darauf richten, die richtigen Stellen der Schleimhaut zu finden und zu treffen. Er kann dabei nicht noch zusätzlich das Auswertegerät beobachten. Insoweit ist eine akustische Meldung vorteilhaft. In gleicher Weise ist es vorteilhaft, wenn das Auswertegerät nach Quittieren einer erfolgreichen Messung durch akustisches Signal den erfaßten Dickenwert der Schleimhaut solange anzeigt, bis eine neue Messung begonnen oder die Messung vollständig abgeschlossen wird. Dadurch kann der Bedienende, im allgemeinen der Zahnarzt, sich vollständig auf den Einsatz der Sonde konzentrieren.

Insgesamt ist die Meßvorrichtung so aufgebaut und ausgelegt, daß das Ultraschallmeßverfahren keiner besonderen Beachtung bedarf, vielmehr das Gerät sich insoweit selbst kontrolliert, daß bei Vorliegen ausreichender Ankopplung und eines vernünftigen Echos von der Oberfläche des Kieferknochens eine Messung durchgeführt wird, sind aber Ankopplung und/oder Echo vom Kieferknochen unzureichend, wird keine Messung durchgeführt, so dar auch keine Fehlmessung anfallen kann.

Das Material für den Vorlaufkörper soll eine Schallimpedanz (Schallhärte) haben, die in Nähe des Wertes der Schallimpedanz von Wasser liegt. Hierdurch wird die erfindungsgemäße deutliche Unterscheidung zwischen einer Ankopplung an eine Schleimhaut und einer Messung gegen Luft ermöglicht. Das Material für den Vorlaufkörper kann wie folgt ausgewählt werden: Bei vollständig sauberer Frontfläche (kein Wassertropfen oder Schmutzpartikel auf dieser) wird die Höhe des ersten Echos (oder eines anderen Echos) der Koppelfläche des Vorlaufkörpers gegen Luft gemessen. Sodann wird unter Beibehaltung aller sonstigen Parameter der Vorlaufkörper mit seiner Frontfläche vollständig in Wasser getaucht. Die Höhe des betrachteten Echos sollte dann kleiner als 20 %, vorzugsweise kleiner als 5 % des gegen Luft gemessenen Wertes sein. Unter diesen Kriterien wird das Material ausgewählt.

Das Material für den Vorlaufkörper sollte auch eine Schallgeschwindigkeit haben, die möglichst gering ist, also möglichst in Nähe der Schallgeschwindigkeit von Wasser liegt. Dadurch kann der Vorlaufkörper kurz ausgebildet werden.

Vorzugsweise muß, um eine gültige Messung durchzuführen, mindestens eine Ankopplung für die Dauer von drei Meßtakten vorliegen. Bleibt die Ankopplung für kürzere Zeit nur bestehen, erfolgt eine Fehleranzeige. Vorzugsweise mittelt das Gerät über mehrere Einzeimeßwerte und gibt den gemittelten Wert zur Anzeigevorrichtung.

Als sehr vorteilhaft hat es sich erwiesen, die Kalibrierungsphase automatisch starten und ablaufen zu lassen, sie wird von der Steuerelektronik ausgelöst. Die Kalibrierungsphase läuft jeweils dann ab, wenn das Gerät neu eingeschaltet wird oder wenn die Steckverbindung des Kabels zwischen Handsonde und Auswertegerät bei eingeschaltetem Auswertegerät hergestellt wird.

Ein vorteilhaftes Ausführungsbeispiel der Meßvorrichtung, das auch zugleich zur Erläuterung des erfindungsgemäßen Meßverfahrens dient und nicht einschränkend zu verstehen ist, wird im folgenden näher beschrieben und unter Bezugnahme auf die Zeichnung erläutert. In dieser zeigen:
- Fig. 1: eine Draufsicht auf das Gesamtgerät
- Fig. 2: einen Schnitt entlang der Schnittlinie II-II in Figur 3 und
- Fig. 3: eine Ansicht entsprechend III-III in Figur 2.

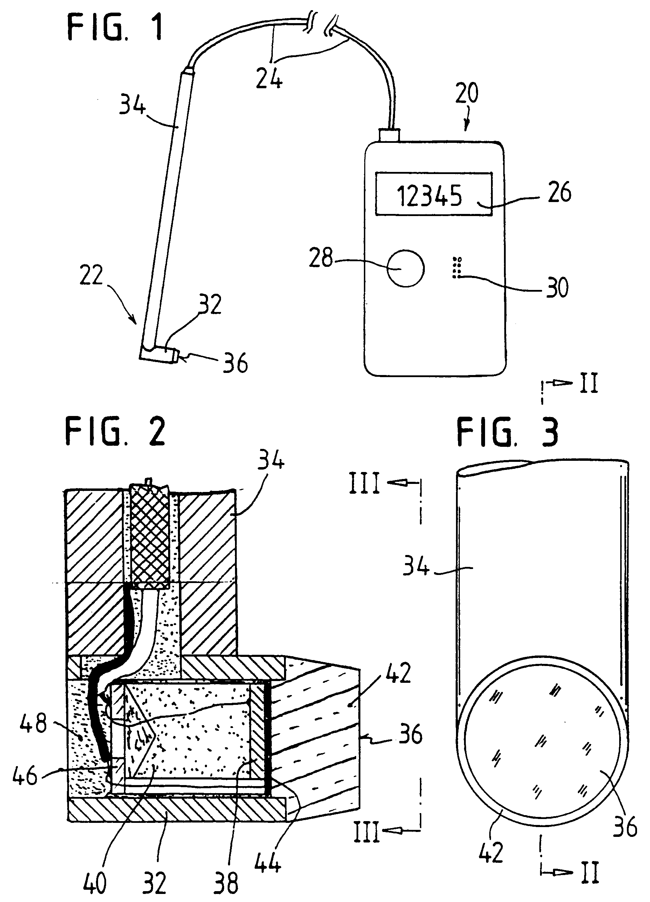

Wie Figur 1 zeigt, besteht die vollständige Meßvorrichtung aus einem handlichen Auswertegerät 20, das etwa handgroß ist, und einer Sonde 22, beide sind über ein Kabel 24 lösbar miteinander verbunden. Im Auswertegerät 20 befindet sich eine Anzeigeeinheit 26 mit LCD (liquid crystal display), weiterhin ein Ein-Ausschalter 28 und (unter Öffnungen verborgen) ein Signalgeber 30. Weiterhin ist im Gehäuse des Auswertegerätes 20 eine Steuer- und eine Auswerteelektronik sowie eine Spannungsversorgung untergebracht.

Die Handsonde 22 besteht aus einem ersten, das untere Ende bildenden Gehäuseteil 32 und einem zweiten Gehäuseteil 34. Das erste Gehäuseteil wird im folgenden noch näher beschrieben, es ist insbesondere aus den Figuren 2 und 3 ersichtlich. Beide Gehäuseteile sind aus Polysulfon-Rohr, weiß, mit 6 mm Außendurchmesser hergestellt. Das zweite Gehäuseteil 34 hat eine Länge von etwa 150 mm, das erste Gehäuseteil 32 ist etwa 10 mm lang. Wie aus Figur 1 ersichtlich, bilden die beiden Gehäuseteile 32 und 34 eine L-förmige Anordnung. Die Stirnfläche des freien Endes des ersten Gehäuseteils 32 ist die Koppelfläche 36, auf sie wird später noch eingegangen. Die beiden Gehäuseteile 32, 34 sind so miteinander verbunden, daß keine Spalte vorliegen, der Innenraum beider Gehäuseteile 32, 34 ist mit einer geeigneten Füllmasse, z. B. Silikon, ausgefüllt.

Im ersten Gehäuseteil 32 ist ein Ultraschallschwinger 38 (Piezoschwinger) rechtwinklig zur Rohrachse des ersten Gehäuseteils 32 angeordnet, er hat einen Durchmesser von etwa 3,3 mm und ist für 5 MHz ausgelegt. Rückwärtig schließt sich an ihn ein Dämpfungskörper 40 an, er hat einen kegelförmigen Einschnitt zur Verhinderung von Reflektionen seiner Rückfläche. An der anderen Schallemissionsfläche des Ultraschallschwingers 38 ist ein Vorlaufkörper 42 angeordnet. Er ist aus einem Polymethylpenten gefertigt. Dieses Material hat eine Schallimpedanz, die in Nähe der Schallimpedanz von Wasser liegt. Es ist weiterhin für medizinische Anwendungen gut geeignet. Wie Figur 2 zeigt, hat der Vorlaufkörper 42 eine im wesentlichen stumpfkeglige Form, insgesamt aber einen Durchmesser auch im Bereich seiner Koppelfläche 36, der größer ist als der Durchmesser des Ultraschallschwingers 38. Die Koppelfläche 36 ist plan. Zwischen Vorlaufkörper 42 und Ultraschallschwinger 38 befindet sich noch eine Anpaßschicht 44. Sie ist metallisch und dient zugleich der Kontaktierung, wie aus Figur 2 ersichtlich ist. Die Rückseite des Ultraschallschwingers 38 ist metallisiert, sie ist über eine Anschlußleitung kontaktiert. Die Anschlußleitungen sind einem Lötstützpunkt 46 zugeführt, der sich auf der Rückseite des Dämpfungskörpers 40 befindet. Von dort ist die Anschlußleitung in Form einer Koaxialleitung weitergeführt. Der Bereich hinter dem Lötstützpunkt 46, in dem sich auch die Anschlußleitungen befinden, ist mit einer Füllmasse 48 so ausgefüllt, daß ein im wesentlichen ebener Abschluß erreicht wird. Das zweite Gehäuseteil 34 nimmt die Anschlußleitung auf, es dient weiterhin als Handhabe für die Handsonde.

Der Sender des Meßgerätes besteht aus einem Spikepulser mit Schalttransistor, dabei wird die Sendespannung mit Hilfe einer Induktivität erzeugt, die mit einem Ladekondensator in Resonanz steht. In einem ersten Takt wird die Induktivität für eine einstellbare Zeit über Schaltelemente an eine Spannung von 5 Volt gelegt. In einem zweiten Takt wird die Induktivität mit dem Ladekondensator parallel geschaltet. Nach einer Viertelperiode dieses Schwingkreises, wenn die Energie aus der Spule in den Kondensator übergegangen ist, wird der Kondensator über einen Dämpfungswiderstand und den Ultraschallschwinger 38 entladen. Damit wird der Ultraschallsendeimpuls erzeugt. Die Sendeenergie ist ungefährt proportional zur in der Spule gespeicherten Energie, diese wiederum wird durch die Zeit bestimmt, mit der die Spannung von 5 Volt angelegt ist. Diese Zeit wird in Schritten von 250 ns gesteuert.

Der Empfänger besteht aus zwei hintereinander geschalteten Verstärkern mit insgesamt ca. 30 dB Verstärkung. Die Ausgänge beider Verstärker sind jeweils mit Komparatoren verbunden. Mit dem ersten Verstärker werden die Echos der Koppelfläche 36 detektiert, mit dem zweiten die Echos der Oberfläche des Kieferknochens.

In der Auswerteelektronik sind zwei Zeittore (Blenden) vorgesehen, nämlich eine Blende für das erste Echo der Koppelfläche 36 und eine Blende für das nachfolgende Echo. Zeitlich liegt die erste Blende zwischen 2,5 und 4,0 µs und die zweite Blende zwischen 4,0 und 7,0 µs nach dem Sendeimpuls während der Kalibrierungsphase. Liegt kein Echosignal über einer Meßschwelle, wird ein erstes Fehlersignal ausgegeben. Liegt nur eines der beiden Echosignale nicht über einer Schwelle, wird ein zweites Fehlersignal ausgegeben. In beiden Fällen wird ein Meßvorgang nicht zugelassen.

Während der Kalibrierungsphase wird die Sendeamplitude sukzessiv so verringert, daß die Amplitude des ersten Echos gerade die Meßschwelle erreicht. Dieser Wert wird für die folgenden Prozeduren abgespeichert. Damit ist die Empfindlichkeit kalibriert.

Mit dieser Sendeamplitude wird anschließend die Verzögerungszeit des Vorlaufs, also insbesondere des Vorlaufkörpers 42, gemessen und gespeichert.

Während der eigentlichen Messung werden im Systemtakt von 128 ms periodisch Sendeimpulse mit dem kalibrierten Wert erzeugt. Die Blenden werden während der Messung für das erste Koppelflächenecho gesetzt auf eine Zeit zwischen der gemessenen Vorlaufzeit und gemessene Vorlaufzeit zuzüglich 0,5 µs und für das zweite Blendenecho auf gemessene Vorlaufzeit zuzüglich 0,5 µs und gemessene Vorlaufzeit zuzüglich 10,75 us. Weiterhin ist noch für die Messung ein Zeittor vorgesehen, in das die Echos von der Knochenoberfläche hineinfallen müssen, es ist so gelegt, daß Schleimhautdicken zwischen 0,5 und 8 mm beispielsweise erfaßt werden können, der Meßbereich kann aber auch auf andere Bereiche ausgedehnt oder eingeschränkt werden.

Vorzugsweise ist für den Meßwerterwartungsbereich, also den Bereich, in den das Echo des Kieferknochen fallen muß, eine Meßschwelle vorgesehen, die so gelegt ist, daß Störsignale nicht zu einer Messung führen können.

In einer nicht dargestellten Ausführung ist der zweite Gehäuseteil 34 biegsam einstellbar, z. B. als Schwanenhals ausgeführt. Es kann daher von Hand gebogen werden, behält aber die eingestellte Verbiegung beim praktischen Einsatz bei.

## Patentansprüche

1. Verfahren zur Messung der Schleimhautdicke eines Kieferknochens, bei dem von einem Ultraschallschwinger nach dem Impuls-Echo-Verfahren Schallimpulse durch einen Vorlaufkörper (42) in die Schleimhaut gesendet und die Schallechos am Kieferknochen erfaßt werden, wobei der Vorlaufkörper (42) aus einem Material gefertigt ist, das eine Schallimpedanz aufweist, die der Schallimpedanz in der Schleimhaut möglichst ähnlich ist und der Vorlaufkörper (42) eine Koppelfläche (36) ausbildet und bei dem in einer Kalibrierphase mindestens ein Echo von der freien, nur an Luft angrenzen den Koppelfläche (36) erfaßt und amplitudenmäßig bewertet wird und während einer Meßphase die Ankopplung der Koppelfläche (36) an die Schleimhaut dadurch überwacht wird, daß dieses Echo von der Koppelfläche (36) deutlich kleiner ist als während der Kalibrierphase, und wobei neben einem Echo von der Koppelfläche (36) noch ein nachfolgendes Echo der Koppelfläche (36) während der Kalibrierphase überwacht wird und bei einem unterhalb eines Schwellenwertes liegenden Verhältnis zwischen dem ersten und dem nachfolgenden Koppelflächenecho ein Error-Signal ausgegeben wird.

2. Verfahren nach Anspruch 1, dadurch gekennzeichnet, daß die dem Ultraschallschwinger periodisch zugesandte elektrische Energie so bemessen wird, daß das Echo der Koppelfläche eine gewisse Amplitude aufweist.

3. Verfahren nach Anspruch 1, dadurch gekennzeichnet, daß ein akustisches Signal ausgegeben wird, wenn eine schlechte Ankopplung vorliegt.

4. Verfahren nach Anspruch 1, dadurch gekennzeichnet, daß eine Messung nur dann durchgeführt wird, wenn mindestens eine Ankopplung für die Dauer von drei Meßtakten vorliegt und daß eine Fehleranzeige ausgegeben wird, wenn die Ankopplung nur für kürzere Zeit besteht.

5. Verfahren nach Anspruch 1, dadurch gekennzeichnet daß die Kalibriesphase automatisch gestartet wird und abläuft jeweils wenn das Gerät neu eingeschaltet wird oder wenn eine Steckverbindung zwischen einer Handsonde und einem Auswertegerät bei eingeschaltetem Auswertegerät hergestellt wird.

## Claims

1. Method for measuring the thickness of a mucous membrane of a jaw-bone, with which acoustic pulses are being transmitted by an ultrasonic oscillator in accordance with the pulse-echo process via a probe lip (42) into the mucous membrane and the acoustic echos being registered at the jaw-bone, and where the probe tip (42) is made from a material, which shows an acoustic impedance, which is as similar to the acoustic impedance in the mucous membrane as possible and the probe tip (42) forming a coupling-surface (36) and with which during calibrating at least one echo of the free coupling-surface (36), being in contact with air, is being seized and evaluated by amplitude and while evaluating the coupling of the coupling-surface (36) to the mucous membrane is controlled by the fact that said echo of the coupling-surface (36) is remarkably smaller than during calibrating, and where additionally to the echo of the coupling-surface (36) a subsequent echo of the coupling-surface (36) during calibrating is being controlled and an error signal is emitted with a ratio below a threshold value between the first and the subsequent echo of the coupling-surface.

2. Procedure according to claim 1 characterized by the fact that the electrical energy periodically transmitted to the ultrasonic oscillator is measured in such a way that the echo of the coupling-surface shows a certain amplitude.

3. Procedure according to claim 1 characterized by the fact that an acoustic signal is sent, if an error occurs while coupling.

4. Procedure according to claim 1 characterized by the fact that it can only be measured if a coupling exists for the period of three measuring units and that an error indication is given, if the coupling exists for a shorter period of time.

5. Procedure according to claim 1 characterized by the fact that the calibrating phase is started and run automatically every time the device is switched on or if a plug connection between a hand-held probe and an evaluator is being formed while the evaluator is supplied with power.

## Revendications

1. Méthode de mesurage de l'épaisseur de la muqueuse d'un os maxillaire, dans la cas de laquelle un oscillateur ultrasonore emet, selon la méthode par écho d'impulsion, des impulsions sonores à travers un avant-corps (42) dans la muqueuse et les échos sonores sont détectés sur l'os maxillaire, l'avant-corps (42) étant fabriqué d'un matériau présentant une impédance sonore qui est dans une large mesure semblable à l'impédance sonore dans la muqeuse et l'avant-corps (42) formant une face de couplage (36), et dans le cas de laquelle du moins un écho est détecté - durant une phase de calibrage - par la face de couplage (36) libre qui n'est contiguë qu'à l'air et est évalué à partir d'amplitudes, et le couplage de la face de couplage (36) avec la muqueuse est surveillé au cours d'une phase de mesurage par le fait que cet écho de la face de couplage (36) est considérablement plus petit que durant la phase de calibrage, et - outre un écho de la face de couplage (36) - un écho suivant de ladite face de couplage (36) étant surveillé durant la phase de calibrage, et un signal d'erreur étant fourni au cas où le rapport entre le premier écho et l'écho suivant de la face de couplage est au-dessous d'une valeur seuil.

2. Méthode selon la revendication 1, caractérisée par le fait que l'énergie électrique fournie de manière périodique à l'oscillateur ultrasonore est telle que l'écho de la face de couplage présente une certaine amplitude.

3. Méthode selon la revendication 1, caractérisée par le fait qu'un signal acoustique est fourni au cas où il y a un mauvais couplage.

4. Méthode selon la revendication 1, caractérisée par le fait qu'un mesurage n'est effectué qu'au cas où il y a un couplage pour la durée de trois temps de mesure du moins, et qu'une indication d'erreur est fournie si le couplage n'existe que pour une plus courte durée.

5. Méthode selon la revendication 1, caractérisée par le fait que la phase de calibrage est déclenchée de manière automatique et se déroule respectivement au cas où l'appareil est de nouveau mis en circuit ou au cas où l'on réalise une jonction mâle et femelle entre une sonde à main et un appareil d'évaluation lorsque l'appareil d'évaluation est en circuit.
